# EUROPÄISCHE PATENTSCHRIFT

(11) **EP 2 429 334 B1**
(45) Veröffentlichungstag und Bekanntmachung des Hinweises auf die Patenterteilung: **13.03.2013**
(21) Anmeldenummer: 10720241.8
(22) Anmeldetag: 11.05.2010
(51) Int. Cl.: A45F 5/02, A61J 17/00

(54) **SCHNULLERBAND-KLAMMER**
BABY'S DUMMY CLIP
PINCE POUR CORDON DE SUCETTE

(30) Priorität: 11.05.2009 AT 7102009
(43) Veröffentlichungstag der Anmeldung: 21.03.2012
(73) Patentinhaber: Mam Babyartikel Gesellschaft m.b.H., 1160 Wien (AT)
(72) Erfinder: RÖHRIG, Peter, A-1160 Wien (AT); BERANEK, Ernst, 1160 Wien (AT)
(74) Vertreter: Sonn & Partner Patentanwälte
(86) Internationale Anmeldenummer: PCT/AT2010/000161
(87) Internationale Veröffentlichungsnummer: WO 2010/138981

(56) Entgegenhaltungen:
- WO-A1-2004/023919
- US-A- 4 489 466
- US-A1- 2003 115 726

## Beschreibung

Die Erfindung betrifft eine Schnullerband-Klammer mit einem im Wesentlichen U-förmigen Profilteil, dessen Scheitel als Schwenkbereich ausgebildet ist und dessen Schenkel als relativ zueinander schwenkbare Klemmteile ausgebildet sind, die zusammenarbeitende Klemmbereiche aufweisen, wobei in einer geschlossenen Klemmstellung der Schnullerband-Klammer einander zugewandte, innenseitige Oberflächen der Klemmbereiche im Wesentlichen aneinander liegen und die Klemmbereiche in ihrer Offenstellung voneinander beabstandet sind, wobei an einem der Klemmteile ein Verschlussteil drehbar gelagert ist, über welchen die Klemmbereiche in ihre Klemmstellung überführbar sind.

Klammern bzw. Clips zur Befestigung von Schnullerbändern oder dergl. an Kleidungsstücken eines Kleinkindes bzw. an anderen Gegenständen, z.B. einem Kinderwagenelement, sind grundsätzlich bereits bekannt.

Aus der US 2003/0115726 A1 ist ein Klammer der eingangs angeführten Art mit zwei zueinander schwenkbaren Klemmteilen bekannt, wobei in einer geschlossenen Klemmstellung einander zugewandte innenseitige Oberflächen von Klemmbereichen aneinander liegen und die Klemmbereiche in einer Offenstellung voneinander beabstandet sind. Zum Überführen der Klemmbereiche in ihre Klemmstellung weist ein Klemmteil einen Gewindestift auf, welcher sich durch eine Öffnung im anderen Klemmteil hindurch erstreckt und auf welchem ein Schraubelement zum Schließen der Klammer aufschraubbar ist. Um beim Verdrehen des Schraubelements in Richtung einer Offenstellung ein automatischen Überführen der Klemmbereiche in eine Offenstellung zu erzielen, muss das Material, aus welchem die Klammer besteht, nachteiligerweise ein hohe Eigenelastizität aufweisen; andernfalls verbleiben die Klemmbereiche auch beim Verdrehen des Schraubelements in Richtung einer Offenstellung in ihrer geschlossenen Stellung.

In der WO 2004/023919 A1 ist eine Schnullerband-Klammer geoffenbart, die einen U-förmigen Profilteil aus einem relativ harten Kunststoffmaterial, etwa Polycarbonat, aufweist. Die Oberfläche der Klemmteile besteht bei dieser Vorrichtung zumindest teilweise aus weicherem Material, wodurch ein verbesserter Halt auf glatten oder dünnen Stoffen gewährleistet ist. Zum Überführen zwischen Offen- und Schließstellung ist eine die beiden Klemmteile umgreifende, auf ihnen verschieblich gelagerte Lasche vorgesehen; durch Verschieben der Lasche in Richtung der frei auskragenden Enden der Klemmteile kann die Klammer somit geschlossen werden. Eine derartige Schließvorrichtung mit einer Lasche funktioniert grundsätzlich zufriedenstellend, jedoch ist zum selbständigen Öffnen der Klammer erforderlich, dass das Material, aus welchem der U-förmige Profilteil besteht, eine hohe Eigenelastizität aufweist. Demzufolge sind bei der Auswahl der Materialien nachteiligerweise enge Grenzen gesetzt.

Aus der DE 35 10 906 A1 ist ein Clip für Hosenträger bekannt, bei dem eine im Wesentlichen als U-Profil ausgebildete Blattfeder zwei Klemmbacken trägt. Hierbei weist die Blattfeder jedoch eine Vorspannung auf, so dass der Hosenträger-Clip in einer nicht-belasteten Stellung in seiner Schließstellung vorliegt und mittels eines Nockens in eine Offenstellung überführt werden muss.

Die US 4,489,466 A offenbart weiters eine andersartige (Hosenträger-)Klammer mit zwei Teilen, die um eine zentrale Schwenkachse relativ zueinander verschwenkbar sind.

Eine weitere Babyschnuller-Klammer ist aus der US 5 948 003 A bekannt. Hier sind zwei gesonderte Klemmteile vorgesehen, die mittels einer Schenkelfeder in ihrer geschlossenen Stellung gehalten werden; ein Mechanismus, der etwa von Wäscheklammern bekannt ist. Zugleich kann diese Klammer als Abdeck- bzw. Schutzelement für den Schnullersauger verwendet werden.

Auch aus der DE 26 18 880 C2 ist eine Klammer mit zwei zueinander schwenkbaren Teilen bekannt, die ohne Feder, Hebel und Stift auskommt. Ein Öffnen bzw. Schließen der Klammer wird durch ein Verschieben eines mit einer Abschrägung versehenen Schiebers erzielt. Hierin liegt ein konstruktiv aufwendiger, unhandlicher Öffnungsmechanismus für die Klemme vor.

Demgegenüber ist es Aufgabe der vorliegenden Erfindung, eine Schnullerband-Klammer zu schaffen, bei welcher auf einfache Weise ein Überführen zwischen einer Offen- und einer Schließstellung möglich ist, wobei ein Überführen in die Offenstellung mit Hilfe des Verschlussteils unabhängig von der Eigenelasitizität des für die Klammer vorgesehen Materials erzielt werden können soll.

Dies wird bei der Schnullerband-Klammer der eingangs angeführten Art dadurch erzielt, dass der Verschlussteil zumindest eine Mitnahmeeinrichtung aufweist, so dass beim Verdrehen des Verschlussteils in Richtung der Offenstellung die beiden Klemmbereiche voneinander entfernt werden. Mit Hilfe der Mitnahmevorrichtung ist somit unabhängig von der Materialwahl für die Klammer, d.h. auch bei Materialien mit einem relativ niedrigen Elastizitätsmodul, zuverlässig ein automatisches Überführen der Klemmbereiche in eine Offenstellung bei entsprechender Verdrehung des Verschlussteils gewährleistet. Demzufolge wird eine einfach zu bedienende, zuverlässig öffnende Klammer geschaffen, bei welcher das Material nicht zwingend eine bestimmte Eigenelastizität aufweisen muss. Eine konstruktiv einfache, geringe Teilezahl aufweisende Ausführung der Klammer wird erzielt, indem ein im Wesentlichen U-förmiger Profilteil vorgesehen ist, dessen Scheitel als Schwenkbereich ausgebildet ist und dessen Schenkel als Klemmteile ausgebildet sind.

Eine konstruktiv einfache, zweckmäßige Vorrichtung zum Mitnehmen eines Klemmteils beim Öffnen des Verschlussteils ist gegeben, wenn die Mitnahmeeinrichtung ein mit dem relativ zu dem das Verschlussteil aufnehmenden Klemmteil schwenkbaren Klemmteil verbundenes Verbindungselement aufweist, das über korrespondierende Verbindungsflächen mit dem drehbar gelagerten Verschlussteil verbunden ist. Beim Öffnen der Klammer nimmt das Verschlussteil über das Verbindungselement das entsprechende Klemmteil mit, so dass die Klemmbereiche zuverlässig voneinander beabstandet werden. Als Verbindungselement kann insbesondere ein umlaufender Flansch mit einer rastförmigen Verbindungsfläche vorgesehen sein, der in eine entsprechende umlaufende Rastfläche des Verschlusselements eingreift. Bei einer alternativen Ausführung ist als Mitnahmeelement zumindest ein an einer Innenseite eines Klemmteils angreifendes, hakenförmiges Element vorgesehen.

Als Material für die Klemmteile wurde in der Vergangenheit bisher häufig Polycarbonat vorgeschlagen, da dieses Material eine geringe Kriechneigung aufweist und somit zuverlässig eine Rückführung des U-Profils in eine entspannte Offenstellung, z.B. beim Verschieben einer Lasche, gewährleistet ist. Ein Maß für die Kriechbeständigkeit eines Materials ist dabei durch den Koeffizienten C_{c} gegeben, welcher Werte zwischen 0 und 1 annehmen kann. Der Koeffizient C_{c} berechnet sich hierbei aus dem Verhältnis der Kriechmodule E_{c3} und E_{c1} für die Belastungszeiten t₃=1000h und t₁=1h, d.h. C_{c} = E_{c3}/E_{c1}. Der Wert 1 beschreibt einen Werkstoff ohne Kriechen; Polycarbonat weist beispielsweise eine Kriechbeständigkeit C_{c} von 0,86 auf. Eine ausführliche Diskussion der Kriechbeständigkeit C_{c} von Kunststoffen kann J. Kurz, "Kriechbeständigkeit - ein Kennwert für das Kriechverhalten", Kunststoffe 1/2004, Carl Hanser Verlag, München entnommen werden. Da eine geringe Kriechneigung bei der erfindungsgemäßen Schnullerband-Klammer nicht zwingend zum Öffnen der Klammer aus ihrer Schließstellung erforderlich ist, kann der U-förmige Profilteil aus einem Material mit einer Kriechbeständigkeit C_{c} kleiner 0,86 bestehen.

Um die Gesundheitsverträglichkeit der Schnullerband-Klammer auch für Säuglinge und Kleinkinder zu gewährleisten, ist es vorteilhaft, wenn der U-förmige Profilteil aus einem Thermoplast, insbesondere Polyester, besteht.

Eine vorteilhafte Ausführung der Klammer sieht vor, dass zwischen dem drehbar gelagerten Verschlussteil und dem Klemmteil eine Gewindeverbindung vorgesehen ist. Durch die besagte Gewindeverbindung ist bei Drehen des Verschlussteils in beide Richtungen eine Veränderung des relativen Abstands zwischen den Klemmbereichen zuverlässig sichergestellt.

Insbesondere ist es vorteilhaft, wenn der den Verschlussteil aufnehmende Klemmteil eine zylinderförmige Aufnahme mit einem vorzugsweise segmentförmigen Innengewinde aufweist, in welches ein zylinderförmiger Gewindeteil des Verschlussteils mit einem vorzugsweise segmentförmigen Außengewinde eingreift. Ein Drehen des Verschlussteils lässt somit das Außengewinde, das an einem zylinderförmigen Gewindeteil des Verschlussteils angeordnet ist, an dem Innengewinde des den Verschlussteil aufnehmenden Klemmteils eingreifen, wodurch der Abstand der beiden Klemmbereiche verändert wird. Wenn das verwendete Gewinde eine hohe Gewindesteigung aufweist, so dass im Wesentlichen mit einer einzigen Umdrehung die Klammer von der Offenstellung in die Schließstellung überführt wird, ist ein Öffnen bzw. Schließen der Klammer auf besonders einfache, benutzerfreundliche Weise möglich.

Um ein ungewolltes Lösen des Verschlussteils aus der zylinderförmigen Aufnahme zu vermeiden, ist es günstig, wenn der relativ zu dem das Verschlussteil aufnehmenden Klemmteil schwenkbare Klemmteil mit zumindest einem insbesondere rampenförmigen Vorsprung verbunden ist, der eine Anschlagfläche aufweist, die in der Offenstellung der Klemmteile an einer entsprechenden Anschlagfläche des Verschlussteils anliegt. Vorzugsweise sind zwei gegenüberliegende, rampenförmige Vorsprünge vorgesehen. Jeder rampenförmige Vorsprung weist eine gegenüber der Haupterstreckungsebene des Verbindungselements geneigte Fläche und eine im Wesentlichen senkrecht zur Haupterstreckungsebene des Verbindungselements angeordnete Anschlagfläche auf, die nach Verdrehen des Verschlussteils um einen bestimmten Öffnungswinkel an der entsprechenden Anschlagfläche des Verschlussteils anliegt, so dass ein weiteres Verdrehen des Verschlussteils verhindert wird. Die Anschlagfläche des Verschlusselements ist vorzugsweise an einem rampenförmigen Vorsprung des Verschlusselements ausgebildet, der an der Innenseite des Verschlusselements angeordnet ist. Beim Verdrehen des Verschlusselements in die Schließstellung kann die geneigte Fläche des rampenförmigen Vorsprungs des Verbindungselements an der korrespondierenden geneigten Fläche des rampenförmigen Vorsprungs des Verschlusselements auflaufen. Um das ungewollte Lösen des Verschlussteils aus der zylinderförmigen Aufnahme zu verhindern, kann alternativ vorgesehen sein, dass das Außengewinde endseitig einen gegenüber einem Gewindegrund erhabenen Zapfen aufweist.

Um ein ungewolltes Öffnen der Klammer in der Schließstellung zu vermeiden, ist es günstig, dass der Verschlussteil ein Bedienelement aufweist, dessen Unterseite in der Klemmstellung an dem dem Klemmteil mit der zylinderförmigen Aufnahme gegenüberliegenden Klemmteil anliegt, wobei an der Unterseite des Bedienteils und der dem Bedienelement zugewandten Oberfläche des Klemmteils ineinander greifende Zähne vorgesehen sind.

Um einen besonders guten Halt der Klammer an einem Kleidungsstück oder dergl. zu gewährleisten, ist eine hohe Steifigkeit der Klammer vorteilhaft. Eine dahingehend günstige Ausführung sieht bei einem der Klemmteile zumindest im Klemmbereich eine bezüglich der innenseitigen Oberfläche der Klemmteile konvexe Krümmung vor, wodurch ein besserer Halt der Klammer erzielt wird.

Die Erfindung wird nachstehend anhand von den in den Zeichnungen dargestellten bevorzugten Ausführungsbeispielen, auf die sie jedoch nicht beschränkt sein soll, noch weiter erläutert. Im Einzelnen zeigen:
Fig. 1 eine perspektivische Ansicht einer Schnullerband-Klammer mit einem U-förmigen Profilteil und einem drehbaren Verschlussteil;
Fig. 2 einen Schnitt gemäß der Linie II-II in Fig. 1;
Fig. 3 eine perspektivische Ansicht der Unterseite des Verschlussteils;
Fig. 4 eine perspektivische Ansicht der Unterseite eines Verschlussteils mit zwei Mitnahmehaken;
Fig. 5 einen Schnitt der Klammer, wie in Fig. 2 gezeigt, wobei jedoch ein Verschlussteil mit Mitnahmehaken vorgesehen ist;
Fig. 6 einen Schnitt der Klammer (ohne Verschlussteil) gemäß der Linie VI-VI in Fig. 5 mit einem konvex gekrümmten Klemmteil;
Fig. 7 eine Schnittansicht einer weiteren Ausführung der Klammer; und
Fig. 8 eine Detailansicht einer Mitnahmeeinrichtung.

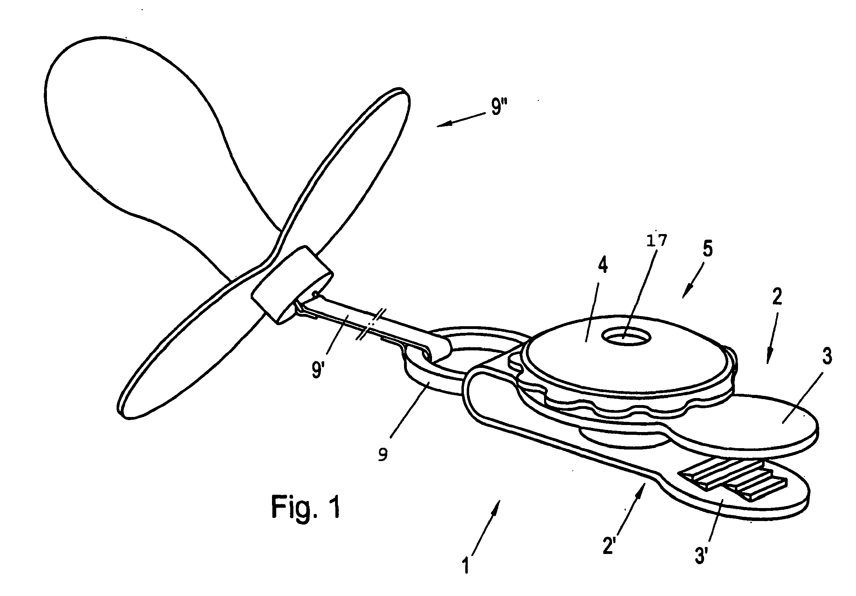

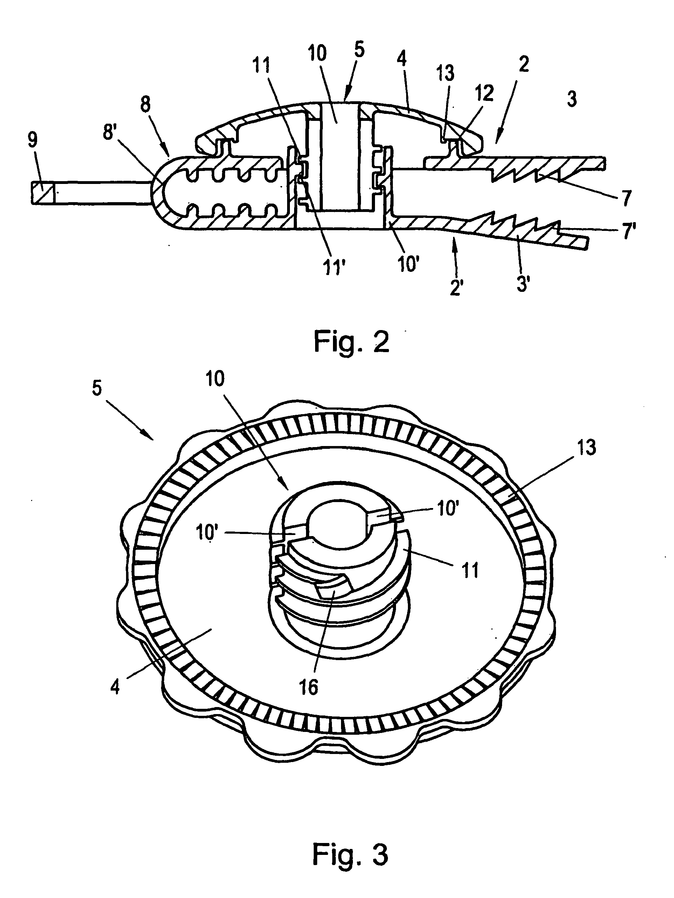

In den Fig. 1 und 2 ist eine Schnullerband-Klammer bzw. ein Clip 1 mit zwei Klemmteilen 2, 2' gezeigt, die jeweils einen Klemmbereich 3, 3' aufweisen. Fig. 1 zeigt die Klemmteile 2, 2' in ihrer Offenstellung, in denen die beiden Klemmbereiche 3, 3' voneinander beabstandet angeordnet sind. Mittels Drehens an einem Bedienelement 4 eines Verschlussteils 5 können die Klemmteile 2, 2' von ihrer Offen- in ihre Schließstellung überführt werden, wobei die beiden Klemmbereiche 3, 3' einander angenähert werden und somit die Klammer 1 an verschiedenen Gegenständen, beispielsweise Kleidungsstücken, einer Seitenwand oder einer Abdeckung eines Kinderwagens oder dergl., befestigt werden kann. Die äußere Kontur des kreisförmigen Bedienteils 4 ist dabei wellenförmig oder geriffelt gestaltet, so dass die Finger in den vertieften Bereichen an dem Bedienelement 4 angreifen können und somit ein guter Halt beim Drehen am Bedienelement 4 erzielt wird. Zudem ist günstig, wenn der Außendurchmesser des kreisförmigen Bedienelements 4 zumindest 39mm aufweist, so dass auf einfache Weise eine Einhandbedienung möglich ist. Das Bedienelement 4 sowie die Klammer 1 weisen eine im Wesentlichen glatte Oberfläche auf, so dass sie auf einfache Weise zu Dekorationszwecken bedruckt werden können.

Wie in Figur 1 dargestellt, ist der Verschlussteil 5 mit einem Ventilationsloch 17 versehen. Aus Norm- und Sicherheitstechnischen Gründen beträgt der Durchmesser des Ventilationslochs 17 zumindest 4 mm, wobei der Lochdurchmesser vorzugsweise kleiner als 5mm oder größer als 12mm gewählt wird, um zu vermeiden, dass ein Finger eines Kindes in dem Ventilationsloch 17 stecken bleibt.

In den Klemmbereichen 3, 3' weisen die einander zugewandten Oberflächen jeweils ein gezahntes Profil 7, 7' auf, wodurch z.B. beim Einklemmen eines dünnen Kleidungsstückes ein verbesserter Halt der Klammer 1 erlangt wird.

Die beiden Klemmteile 2, 2' sind als Schenkel eines U-Profils 8 (vgl. Fig. 2) ausgebildet, wobei ein Scheitelbereich 8' des U-Profils 8 als Schwenkbereich für die beiden Klemmteile 2, 2' ausgebildet ist. Im Scheitelbereich 8' ist zudem ein die Form eines Teils eines Kreisrings aufweisender Befestigungsring 9 vorgesehen, an welchem ein Schnullerband 9' mit einem Schnuller 9'' angebracht werden kann. Das mit dem Befestigungsring 9 verbundene Ende des Schnullerbandes 9', welches wie in Figur 1 dargestellt als Schlaufe ausgebildet sein kann, ist vorteilhafterweise frei gegenüber dem Befestigungsring 9 verschiebbar, so dass die Beweglichkeit des Schnullerbandes 9 nicht durch die Befestigung an der Klammer 1 eingeschränkt wird. Aus Sicherheitsgründen weist auch der Befestigungsring 9 einen Innendurchmesser von zumindest 12mm auf, um die Verletzungsgefahr durch Einklemmen von Fingern zu vermeiden.

Wie insbesondere in Fig. 2 ersichtlich, ist ein zylinderförmiger Gewindeteil 10 des Verschlussteils 5 mit einem Außengewinde 11 in einem Innengewinde 11', das in einer zylinderförmigen Aufnahme 10' des unteren Klemmteils 2' ausgebildet ist, aufgenommen. Je nach Drehrichtung des Verschlussteils 5 werden die beiden Schenkel des U-Profils 8, d.h. die Klemmteile 2, 2', durch Anlage des Bedienteils 4 am oberen Klemmteil 2 somit einander angenähert oder jedoch ein Überführen in die geöffnete Ausgangsstellung ermöglicht. Die beiden Gewinde 11, 11' weisen jeweils eine hohe Gewindesteigung auf, so dass weniger als eine einzige bzw. maximal eine einzige Umdrehung erforderlich ist, um die Klammer 1 von der Offenstellung in die Schließstellung zu überführen.

Wie insbesondere in Fig. 3 ersichtlich, weist der zylinderförmige Gewindeteil 10 des Verschlussteils 5 zwei diametral angeordnete, in Längsrichtung des Zylinders verlaufende schlitzförmige Öffnungen 10" auf. Hierdurch kann der Gewindeteil 10 komprimiert und in die Aufnahme 10' eingesetzt werden. Endseitig weist der Gewindeteil 10 einen gegenüber einem Gewindegrund 16' erhabenen Zapfen 16 auf, welcher ein ungewolltes Herauslösen des Verschlussteils 5 aus der Aufnahme 10' verhindert, indem er in einer nicht komprimierten Stellung des Gewindeteils 10 an einer Flanke des Innengewindes 11' anschlägt, sofern der Verschlussteil 5 in seiner Offenstellung angeordnet ist. Das Außengewinde 11 ist hierbei als zweigängiges Gewinde ausgebildet, um bei der vergleichsweise großen Gewindesteigung die übertragenen Kräfte zu erhöhen.

Wie aus Fig. 2 und 3 ersichtlich ist, wird der Verschlussteil 5 in seiner Schließstellung gehalten (d.h gegen ein ungewolltes Rückverdrehen blockiert), indem an der Unterseite des Bedienteils 4 und der dem Bedienelement 4 zugewandten Oberfläche des Klemmteils 2 ineinander greifende Zähne 12, 13 vorgesehen sind.

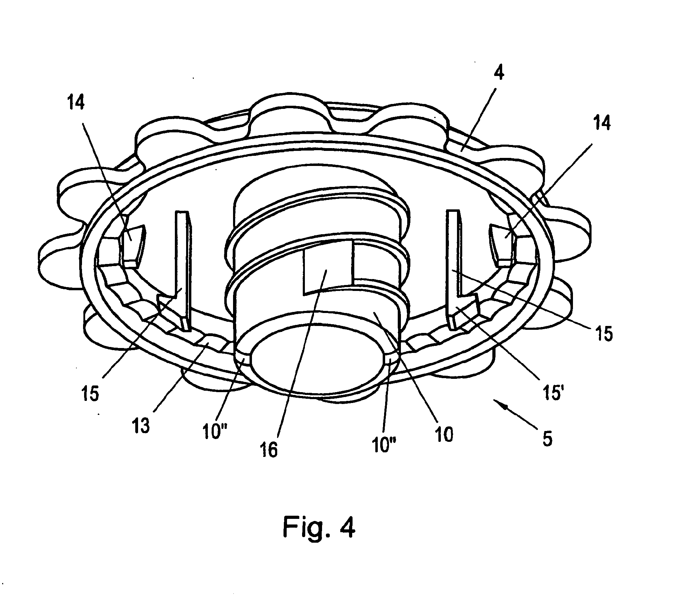

Wie in Fig. 4 ersichtlich, können an der Unterseite des Bedienteils 4, benachbart eines von den Zähnen 13 gebildeten Zahnkranzes, Zapfen 14 vorgesehen sein, die in der Schließstellung an einem entsprechenden Gegenelement des U-Profils 8 (nicht gezeigt) anschlagen und somit ein weiteres Verdrehen des Verschlussteils 5 verhindern; ein überhöhtes Anziehen des Verschlussteils 5 und somit der Bruch der Gewindeaufnahme 10' kann somit verhindert werden.

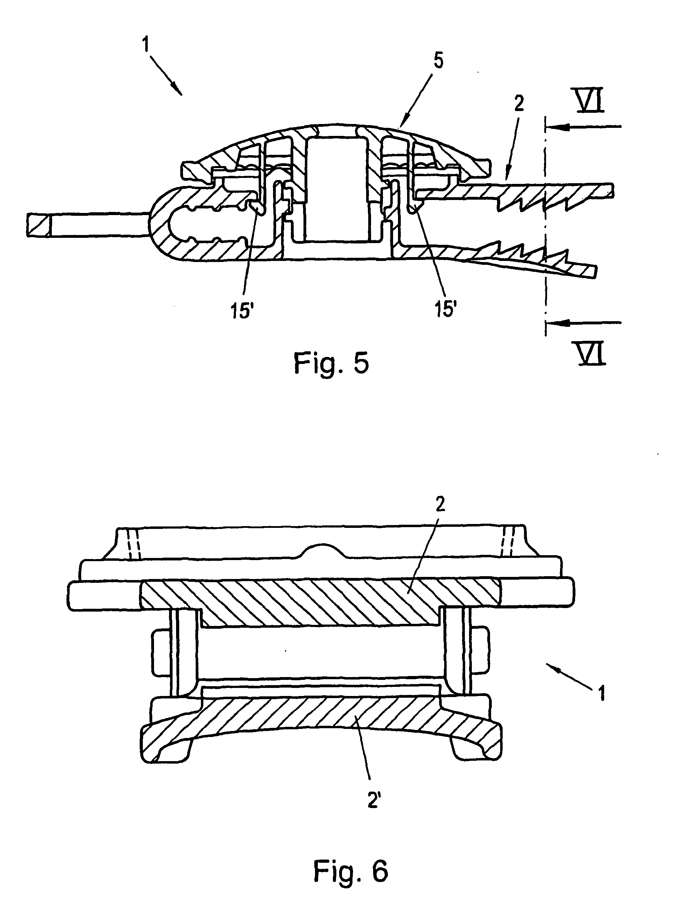

In Fig. 4 und 5 sind weiters zwei Mitnahmeelemente 15 ersichtlich, welche mit ihren hakenförmigen Vorsprüngen 15' den Klemmteil 2 hintergreifen. Während des Öffnungsvorgangs liegen die hakenförmigen Vorsprünge 15' der Mitnahmeelemente 15 somit an einer Innenseite des Klemmteils 2 an, so dass bei Verschiebung des Verschlussteils 5 durch Drehung die Klemmteile 2, 2' automatisch auseinander gezogen werden. Hierdurch ist vorteilhafterweise gewährleistet, dass auch bei Verwendung eines Kunststoffmaterials für das U-Profil 8, das eine vergleichsweise geringe Elastizität aufweist, d.h. z.B. von Polyester oder von anderen, eine sehr gute Gesundheitsverträglichkeit aufweisenden Kunststoffen, auch nach langem Verbleiben in der Schließstellung ein Öffnen der Klemmteile 2, 2' durch Verdrehen des Verschlussteils 5 gewährleistet ist, ohne dass der Benutzer eine gesonderte Zugkraft auf die Klemmteile 2, 2' aufbringen muss, um diese voneinander zu entfernen.

In Fig. 6 ist noch ersichtlich, dass der untere Klemmteil 2' eine bezüglich der zueinander zugewandten Innenseiten der Klemmteile 2, 2' konvexe Krümmung aufweist, wodurch eine höhere Steifigkeit und damit einhergehend ein besserer Halt der Klammer 1 an verschiedenen Gegenständen erzielt werden kann.

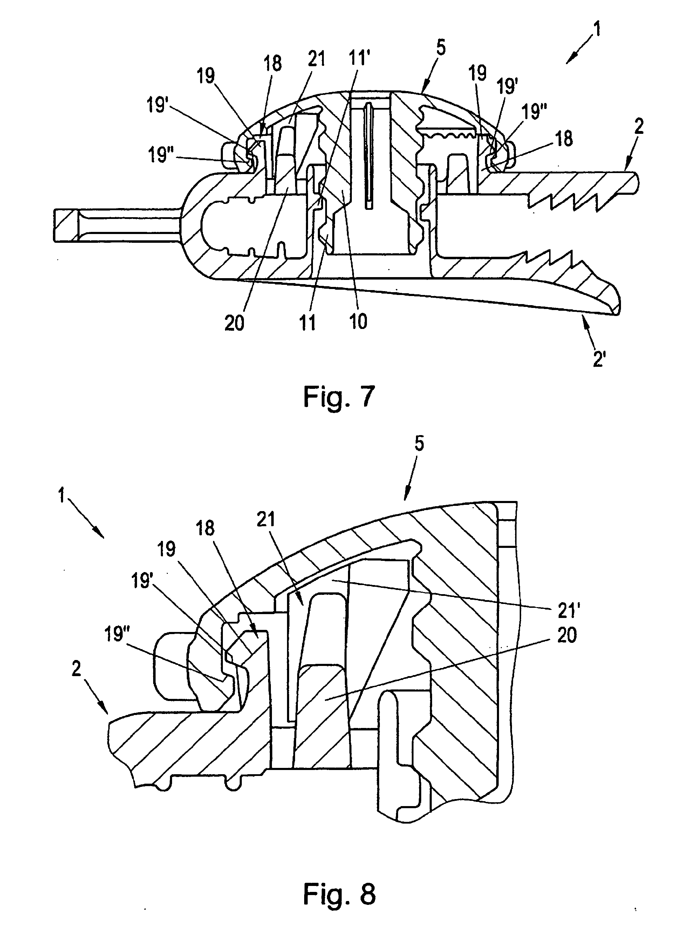

Die Fig. 7 und 8 zeigen eine weitere Ausführung der Klammer 1, bei der als Mitnahmeeinrichtung 15 ein mit dem relativ zu dem das Verschlussteil aufnehmenden Klemmteil 2' schwenkbaren Klemmteil 2 verbundenes Verbindungselement 18 vorgesehen ist. Das Verbindungselement 18 ist durch einen einstückig mit dem Klemmteil 2 verbundenen, umlaufenden Flansch 19 gebildet, der eine rastförmige Verbindungsfläche 19' für eine korrespondierende Verbindungsfläche 19'' des Verschlussteils 5 aufweist. Die ineinandergreifenden Verbindungsflächen 19', 19'' gewährleisten dabei die erforderliche drehbare Lagerung des Verschlussteils 5 gegenüber dem Verbindungselement 18. Beim Verdrehen nimmt das Verschlussteil 5 aufgrund der formschlüssigen Verbindung mit dem Verbindungsteil 18 das Klemmteil 2 mit, so dass die Klemmbereiche 3, 3' voneinander beabstandet werden.

Wie aus Fig. 7 und Fig. 8 weiters ersichtlich, ist das Klemmteil 2 mit zwei an gegenüberliegenden Stellen angeordneten rampenförmigen Vorsprüngen 20 verbunden. Die Vorsprünge 20 weisen jeweils eine Anschlagfläche 20' auf, die in der gezeigten Offenstellung der Klemmteile 2, 2' an einer entsprechenden Anschlagfläche 21' eines ebenfalls rampenförmigen Vorsprungs 21 anliegt, der mit der Innenseite des Verschlussteils 5 verbunden ist. Die rampenförmigen Vorsprünge 20, 21 weisen zudem geneigte Flächen auf, die bei Erreichen der Schließstellung der Klammer 1 aneinander auflaufen.

## Patentansprüche

1. Schnullerband-Klammer (1) mit einem im Wesentlichen U-förmigen Profilteil (8), dessen Scheitel (8') als Schwenkbereich ausgebildet ist und dessen Schenkel als relativ zueinander schwenkbare Klemmteile (2, 2') ausgebildet sind, die zusammenarbeitende Klemmbereiche (3, 3') aufweisen, wobei in einer geschlossenen Klemmstellung der Schnullerband-Klammer (1) einander zugewandte, innenseitige Oberflächen der Klemmbereiche (3, 3') im Wesentlichen aneinander liegen und die Klemmbereiche (3, 3') in ihrer Offenstellung voneinander beabstandet sind, wobei an einem der Klemmteile (2, 2') ein Verschlussteil (5) drehbar gelagert ist, über welchen die Klemmbereiche (3, 3') in ihre Klemmstellung überführbar sind, **dadurch gekennzeichnet, dass** der Verschlussteil (5) zumindest eine Mitnahmeeinrichtung (15) aufweist, so dass beim Verdrehen des Verschlussteils (5) in Richtung der Offenstellung die beiden Klemmbereiche (3, 3') voneinander entfernt werden.

2. Schnullerband-Klammer nach Anspruch 1, **dadurch gekennzeichnet, dass** die Mitnahmeeinrichtung (15) ein mit dem relativ zu dem das Verschlussteil (5) aufnehmenden Klemmteil (2') schwenkbaren Klemmteil (2) verbundenes Verbindungselement (18) aufweist, das über korrespondierende Verbindungsflächen (19', 19'') mit dem drehbar gelagerten Verschlussteil (5) verbunden ist.

3. Schnullerband-Klammer nach Anspruch 1 oder 2, **dadurch gekennzeichnet, dass** der U-förmige Profilteil (8) aus einem Material mit einer Kriechbeständigkeit C_{c} kleiner 0,86 besteht.

4. Schnullerband-Klammer nach einem der Ansprüche 1 bis 3, **dadurch gekennzeichnet, dass** der U-förmige Profilteil (8) aus einem Thermoplast, insbesondere Polyester, besteht.

5. Schnullerband-Klammer nach einem der Ansprüche 1 bis 4, **dadurch gekennzeichnet, dass** zwischen dem drehbar gelagerten Verschlussteil (5) und dem Klemmteil (2') eine Gewindeverbindung vorgesehen ist.

6. Schnullerband-Klammer nach einem der Ansprüche 1 bis 5, **dadurch gekennzeichnet, dass** der den Verschlussteil (5) aufnehmende Klemmteil (2') eine zylinderförmige Aufnahme (10') mit einem vorzugsweise segmentförmigen Innengewinde (11') aufweist, in welches ein zylinderförmiger Gewindeteil (10) des Verschlussteils (5) mit einem vorzugsweise segmentförmigen Außengewinde (11) eingreift.

7. Schnullerband-Klammer nach Anspruch 6, **dadurch gekennzeichnet, dass** der relativ zu dem das Verschlussteil (5) aufnehmenden Klemmteil (2') schwenkbare Klemmteil (2) mit zumindest einem insbesondere rampenförmigen Vorsprung (20) verbunden ist, der eine Anschlagfläche (20') aufweist, die in der Offenstellung der Klemmteile (2, 2') an einer entsprechenden Anschlagfläche (21') des Verschlussteils (5) anliegt.

8. Schnullerband-Klammer nach einem der Ansprüche 1 bis 7, **dadurch gekennzeichnet, dass** der Verschlussteil (5) ein Bedienelement (4) aufweist, dessen Unterseite in der Klemmstellung an dem dem Klemmteil (2') mit der zylinderförmigen Aufnahme gegenüberliegenden Klemmteil (2) anliegt, wobei an der Unterseite des Bedienelement (4) und der dem Bedienelement (4) zugewandten Oberfläche des Klemmteils (2) ineinander greifende Zähne (12), (13) vorgesehen sind.

9. Schnullerband-Klammer nach einem der Ansprüche 1 bis 8, **dadurch gekennzeichnet, dass** einer der Klemmteile (2, 2') zumindest im Klemmbereich eine bezüglich der innenseitigen Oberfläche der Klemmteile (2, 2') konvexe Krümmung aufweist.

## Claims

1. A pacifier strap clip (1) comprising a substantially U-shaped profile part (8) the apex (8') of which is formed as a pivoting region and the legs of which are formed as clamping parts (2, 2') which can be pivoted in relation to each other and have co-operating clamping regions (3, 3'), wherein, in a closed clamping position of the pacifier strap clip (1), opposing, inner-side surfaces of the clamping regions (3, 3') substantially rest against each other and, in its open position, the clamping regions (3, 3') are spaced apart from each other, wherein a closure part (5) by means of which the clamping regions (3, 3') can be transferred into their clamping position, is rotatably mounted on one of the clamping parts (2, 2'), **characterized in that** the closure part (5) has at least one engaging device (15) so that, when the closure part(5) is turned in the direction of the open position, the two clamping regions (3, 3') are moved away from each other.

2. The pacifier strap clip according to claim 1, **characterized in that** the engaging device (15) comprises a connecting element (18) connected with the clamping part (2) which can be pivoted in relation to the clamping part (2') receiving the closure part (5), said connecting element (18) being connected with the rotatably mounted closure part (5) via corresponding connecting surfaces (19', 19'').

3. The pacifier strap clip according to claim 1 or 2, **characterized in that** the U-shaped profile part (8) consists of a material with a creep resistance C_{c} of less than 0.86.

4. The pacifier strap clip according to anyone of claims 1 to 3, **characterized in that** the U-shaped profile part (8) consists of a thermoplast, in particular polyester.

5. The pacifier strap clip according to anyone of claims 1 to 4, **characterized in that** between the rotatably mounted closure part (5) and the clamping part (2') there is provided a threaded connection.

6. The pacifier strap clip according to anyone of claims 1 to 5, **characterized in that** the clamping part (2') receiving the closure part (5) comprises a cylindrical receiving element (10') with a preferably segment-shaped female thread (11') into which a cylindrical threaded portion (10) of the closure part (5) with a preferably segment-shaped male thread (11) engages.

7. The pacifier strap clip according to claim 6, **characterized in that** the clamping part (2) which can be pivoted in relation to the clamping part (2') receiving the closure part (5) is connected with at least one, in particular ramp-shaped projecting part (20) which has a stop face (20') which, in the open position of the clamping parts (2, 2'), abuts at a corresponding stop face (21') of the closure part (5).

8. The pacifier strap clip according to anyone of claims 1 to 7, **characterized in that** the closure part (5) comprises an operating element (4) the bottom side of which, in the clamping position, abuts at the clamping part (2) lying opposite the clamping part (2') comprising the cylindrical receiving element, wherein at the bottom side of the operating element (4) and on the surface of the clamping part (2) facing the operating element (4) meshing teeth (12), (13) are provided.

9. The pacifier strap clip according to anyone of claims 1 to 8, **characterized in that** one of the clamping parts (2, 2') at least in the clamping region has a curvature being convex with respect to the inner-side surface of the clamping parts (2, 2').

## Revendications

1. Pince pour cordon de sucette (1) comprenant une partie profilée (8) sensiblement en U, dont le sommet (8') est conçu sous forme de zone de pivotement et dont les côtés sont conçus sous forme de parties de serrage (2, 2') pouvant basculer l'une par rapport à l'autre, qui présentent des zones de serrage (3, 3') coopérantes, sachant que des surfaces, tournées les unes vers les autres dans une position de serrage fermée de la pince pour cordon de sucette (1), des zones de serrage (3, 3') sont disposées sensiblement les unes contre les autres et les zones de serrage (3, 3') sont espacées les unes des autres dans leur position ouverte, une partie de fermeture (5) étant montée de façon rotative sur l'une des parties de serrage (2, 2'), partie de fermeture par laquelle les zones de serrage (3, 3') peuvent être transférées dans leur position de serrage, **caractérisée en ce que** la partie de fermeture (5) présente au moins un dispositif d'entraînement (15), de sorte que les deux zones de serrage (3, 3') sont espacées l'une de l'autre lors de la rotation de la partie de fermeture (5) en direction de la position ouverte.

2. Pince pour cordon de sucette selon la revendication 1, **caractérisée en ce que** le dispositif d'entraînement (15) présente un élément de liaison (18) relié à la partie de serrage (2) basculante par rapport à la partie de serrage (2') recevant la partie de fermeture (5), lequel élément de liaison est relié au moyen de surfaces de liaison (19, 19', 19") correspondantes à la partie de fermeture (5) montée de façon rotative.

3. Pince pour cordon de sucette selon la revendication 1 ou 2, **caractérisée en ce que** la partie profilée (8) en U est à base d'un matériau présentant une résistance au fluage C_{c} inférieure à 0,86.

4. Pince pour cordon de sucette selon l'une des revendications 1 à 3, **caractérisée en ce que** la partie profilée (8) en U est à base d'une matière thermoplastique, en particulier de polyester.

5. Pince pour cordon de sucette selon l'une des revendications 1 à 4, **caractérisée en ce qu'**une liaison filetée est prévue entre la partie de fermeture (5) montée de façon rotative et la partie de serrage (2').

6. Pince pour cordon de sucette selon l'une des revendications 1 à 5, **caractérisée en ce que** la partie de serrage (2') recevant la partie de fermeture (5) présente un logement (10') de forme cylindrique avec un filetage interne (11') de préférence sous forme de segment, filetage dans lequel une partie filetée (10) de forme cylindrique de la partie de fermeture (5) s'engage avec un filetage externe (11) de préférence en forme de segment.

7. Pince pour cordon de sucette selon la revendication 6, **caractérisée en ce que** la partie de serrage (2) pouvant basculer par rapport à la partie de serrage (2') recevant la partie de fermeture (5) est reliée à au moins une saillie (20) en particulier en forme de rampe, laquelle saillie présente une surface de butée (20'), qui s'applique, dans la position ouverte des parties de serrage (2, 2'), sur une surface de butée (21') correspondante de la partie de fermeture (5).

8. Pince pour cordon de sucette selon l'une des revendications 1 à 7, **caractérisée en ce que** la partie de fermeture (5) présente un élément de commande (4), dont le côté inférieur s'applique dans la position de serrage sur la partie de serrage (2) faisant face à la partie de serrage (2') avec le logement de forme cylindrique, sachant que des dents (12), (13), s'engrenant les unes dans les autres sur le côté inférieur de l'élément de commande (4) et de la surface, associée à l'élément de commande (4), de la partie de serrage (2) sont prévues.

9. Pince pour cordon de sucette selon l'une des revendications 1 à 8, **caractérisée en ce que** l'une des pièces de serrage (2, 2') présente au moins dans la zone de serrage une courbure convexe par rapport à la surface côté intérieur des parties de serrage (2, 2').
